(19) Europäisches Patentamt
European Patent Office
Office européen des brevets

(11) Veröffentlichungsnummer : **0 338 330 B1**

(12) **EUROPÄISCHE PATENTSCHRIFT**

(45) Veröffentlichungstag der Patentschrift :
**24.06.92 Patentblatt 92/26**

(51) Int. Cl.⁵ : **C07C 233/45,** C07C 231/08

(21) Anmeldenummer : **89106022.0**

(22) Anmeldetag : **06.04.89**

(54) **Verfahren zur Herstellung von N-Acylaminosäuren.**

(30) Priorität : **16.04.88 DE 3812737**

(43) Veröffentlichungstag der Anmeldung :
**25.10.89 Patentblatt 89/43**

(45) Bekanntmachung des Hinweises auf die Patenterteilung :
**24.06.92 Patentblatt 92/26**

(84) Benannte Vertragsstaaten :
**BE CH DE FR GB IT LI NL**

(56) Entgegenhaltungen :
**EP-A- 0 207 580**
**DE-A- 2 115 985**
**DE-A- 3 242 374**

(73) Patentinhaber : **HOECHST AKTIENGESELLSCHAFT**
**Postfach 80 03 20**
**W-6230 Frankfurt am Main 80 (DE)**

(72) Erfinder : **Jägers, Erhard, Dr.**
**Hemberger Strasse 75**
**W-5303 Bornheim (DE)**
Erfinder : **Koll, Hans-Peter**
**Zur Alten Schmiede 7**
**W-5030 Hürth (DE)**

**EP 0 338 330 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur katalytischen Herstellung von N-Acylaminosäuren durch Umsetzung eines phenyl-substituierten oder unsubstituierten aliphatischen Aldehyds mit einem aliphatischen oder aromatischen Carbonsäureamid und Kohlenmonoxid in Gegenwart eines dipolaren aprotischen Lösemittels.

Ein derartiges Verfahren, welches der Reaktionsgleichung

$$R - CHO + NH_2COR' + CO \xrightarrow{\text{Katalysator}} R - \underset{\displaystyle HN - COR'}{\underset{|}{CH}} - COOH$$

folgt, wurde zuerst durch H. Wakamatsu et al., Chemical Communications, 1971, Seite 1540, bekannt. Die Umsetzung wird in Gegenwart von Wasserstoffgas im Mol-Verhältnis CO : $H_2$ = 3 : 1 beschrieben, obwohl nach der Reaktionsgleichung die Anwesenheit von Wasserstoff nicht vorgesehen ist. Als Katalysator wird Cobaltcarbonyl $Co_2(CO)_8$ in einer Konzentration von 30 mmol Co-Metall je Liter Reaktionsgemisch eingesetzt.

Dasselbe Verfahren, ebenfalls in Gegenwart von Wasserstoffgas und unter zusätzlicher Verwendung einer eine Sulfoxidgruppe enthaltenden Verbindung als Promotor, beschreibt die EP-A-0 170 830. Der Cobaltkatalysator wird dort in einer Konzentration von 100 mmol Co-Metall je Liter Reaktionsgemisch eingesetzt.

Da die verhältnismäßig großen Katalysatormengen erhebliche Schwierigkeiten bei ihrer Abtrennung vom ausreagierten Reaktionsgemisch verursachten, stellte sich die Aufgabe, die Reaktion mit einem wirksameren Katalysator als $Co_2(CO)_8$ durchzuführen.

Überraschenderweise wurde gefunden, daß als Katalysator ein Gemisch aus einer Palladiumverbindung und einem ionischen Halogenid besonders wirksam ist.

Darüberhinaus ist das Verfahren der Erfindung wahlweise und bevorzugt dadurch gekennzeichnet, daß man

a) die Palladiumverbindung, berechnet auf Palladiummetall, in einer Konzentration von 2 - 10 mmol je Liter Reaktionsgemisch einsetzt;
b) das ionische Halogenid in einer Konzentration von 0,05 - 0,5 mol je Liter Reaktionsgemisch einsetzt;
c) als ionisches Halogenid ein Bromid einsetzt;
d) als Palladiumverbindung einen Palladium-Phosphin-Komplex einsetzt.

Während die bekannten Kobaltkatalysatoren in Abwesenheit von Wasserstoff eine erheblich geringere Aktivität zeigen, benötigt das erfindungsgemäß einzusetzende Katalysatorgemisch die Anwesenheit von Wasserstoff nicht, es wurde vielmehr in vielen Fällen sogar eine Hemmung der Reaktionsgeschwindigkeit durch Wasserstoffgas festgestellt.

Als Palladium-Phosphin-Komplex hat sich Bis-Triphenylphosphin-Palladium(II)-chlorid - $PdCl_2[P(C_6H_5)_3]_2$ - besonders bewährt. Darüberhinaus können als Palladiumverbindung z.B. $PdCl_2$, $PdBr_2$ oder Palladium-II-acetat eingesetzt werden.

Als ionisches Halogenid kommen besonders Phosphoniumbromide, z.B. Tetrabutylphosphoniumbromid, sowie Ammonium-, Lithium-, Natrium- und Kaliumbromid in Frage.

Als dipolare aprotische Lösemittel sind bevorzugt einsetzbar: Dioxan, Tetrahydrofuran, Dibutylether, Ethylenglykoldimethylether, Essigsäureethylester, Acetonitril, Sulfolan, N-Methylpyrrolidon oder N,N-Dimethylacetamid.

Die Reaktion kann bei Drucken von 40 bis 200 bar, vorzugsweise bei 80 bis 150 bar, und bei Temperaturen von 20 bis 160°C, vorzugsweise bei 80 bis 140°C, durchgefuhrt werden.

Beispiel 1 (Vergleichsbeispiel nach dem Stand der Technik)

6,0 g Isovaleraldehyd, 4,13 g Acetamid, 70 ml 1,4-Dioxan und 0,42 g $Co_2(CO)_8$ (= 31 mmol Co/l Reaktionsgemisch) werden in einem 150-ml-Autoklaven mit 120 bar CO/$H_2$ im Volumenverhältnis 3 : 1 bei 120°C umgesetzt. Nach einer Reaktionszeit von 60 min wird das Gemisch mittels Hochdruckflüssigchromatographie (HPLC) analysiert. Man findet 6,8 g N-Acetyl-leucin; dies entspricht einer Ausbeute von 55,9 %.

Beispiel 2 (Vergleichsbeispiel nach dem Stand der Technik)

6,0 g Isovaleraldehyd, 4,13 g Acetamid, 70 ml 1,4-Dioxan und 0,42 g $Co_2(CO)_8$ werden in einem 150-ml-Autoklaven mit 120 bar CO bei 120°C umgesetzt. Nach einer Reaktionszeit von 60 min wird das Gemisch mittels HPLC analysiert. Man findet 5,5 g N-Acetyl-leucin; dies entspricht einer Ausbeute von 45,6 %.

Beispiel 3 (Vergleichsbeispiel)

6,0 g Isovaleraldehyd, 4,13 g Acetamid, 70 ml 1,4-Dioxan und 0,25 g Bis(triphenylphosphin)-palladium(II)-chlorid (= 4,4 mmol Pd/l Reaktionsgemisch) werden in einem 15o-ml-Autoklaven mit 120 bar $CO/H_2$ im Volumenverhältnis 3 : 1 bei 120°C umgesetzt. Nach einer Reaktionszeit von 60 min wird das Gemisch mittels HPLC analysiert. Man findet 0,63 g N-Acetyl-leucin; dies entspricht einer Ausbeute von 5,2 %.

Beispiel 4 (Vergleichsbeispiel)

6,0 g Isovaleraldehyd, 4,13 g Acetamid, 70 ml 1,4-Dioxan, 0,25 g Bis(triphenylphosphin)-palladium(II)-chlorid und 4,17 g Tetrabutylphosphoniumbromid (0,15 mol/l Reaktionsgemisch) werden in einem 150-ml-Autoklaven mit 120 bar $CO/H_2$ im Volumenverhältnis 3 : 1 bei 120°C umgesetzt. Nach einer Reaktionszeit von 60 min wird das Gemisch mittels HPLC analysiert. Man findet 6,0 g N-Acetyl-leucin; dies entspricht einer Ausbeute von 49,5 %.

Beispiel 5

6,0 g Isovaleraldehyd, 4,13 g Acetamid, 70 ml 1,4-Dioxan, 0,25 g Bis(triphenylphosphin)-palladium(II)-chlorid und 4,17 g Tetrabutylphosphoniumbromid werden in einem 150-ml-Autoklaven mit 120 bar CO bei 120°C umgesetzt. Nach einer Reaktionszeit von 60 min wird das Gemisch mittels HPLC analysiert. Man findet 8,4 g N-Acetyl-leucin; dies entspricht einer Ausbeute von 69,2 %.

Beispiel 6

6,0 g Isovaleraldehyd, 4,13 g Acetamid, 70 ml 1,4-Dioxan, 0,25 g Bis(triphenylphosphin)-palladium(II)-chlorid und 4,17 g Tetrabutylphosphoniumbromid werden in einem 150-ml-Autoklaven mit 120 bar CO bei 120°C umgesetzt. Nach einer Reaktionszeit von 360 min wird das Gemisch mittels HPLC analysiert. Man findet 9,7 g N-Acetyl-leucin; dies entspricht einer Ausbeute von 79,7 %.

Beispiel 7

9,4 g 3-Phenyl-propionaldehyd, 4,13 g Acetamid, 70 ml 1,4-Dioxan, 0,25 g Bis(triphenylphosphin)-palladium(II)-chlorid und 4,17 g Tetrabutylphosphoniumbromid werden in einem 150-ml-Autoklaven mit 120 bar CO bei 120°C umgesetzt. Nach einer Reaktionszeit von 60 min wird das Gemisch mittels HPLC analysiert. Man findet 10,1 g N-Acetyl-Homophenylalanin; dies entspricht einer Ausbeute von 64,0 %.

Beispiel 8

8,4 g Phenylacetaldehyd, 4,13 g Acetamid, 70 ml 1,4-Dioxan, 0,25 g Bis(triphenylphosphin)-palladium(II)-chlorid und 4,17 g Tetrabutylphosphoniumbromid werden in einem 150-ml-Autoklaven mit 120 bar CO bei 120°C umgesetzt. Nach einer Reaktionszeit von 360 min wird das Gemisch mittels HPLC analysiert. Man findet 3,6 g N-Acetyl-phenylalanin; dies entspricht einer Ausbeute von 25,0 %.

Beispiel 9

5,05 g Isobutyraldehyd, 4,13 g Acetamid, 70 ml 1,4-Dioxan, 0,25 g Bis(triphenylphosphin)-palladium(II)-chlorid und 4,17 g Tetrabutylphosphoniumbromid werden in einem 150-ml-Autoklaven mit 120 bar CO bei 120°C umgesetzt. Nach einer Reaktionszeit von 60 min wird das Gemisch mittels HPLC analysiert. Man findet 7,6 g N-Acetyl-valin; dies entspricht einer Ausbeute von 68,2 %.

Beispiel 10

6,0 g Isovaleraldehyd, 4,13 g Acetamid, 70 ml N-Methylpyrrolidon, 0,25 g Bis(triphenylphosphin)-palladium(II)-chlorid und 2,1 g Lithiumbromid (0,3 mol/l Reaktionsgemisch) werden in einem 150-ml-Autoklaven mit 120 bar CO bei 120°C umgesetzt. Nach einer Reaktionszeit von 60 min wird das Gemisch mittels HPLC analysiert. Man findet 10,9 g N-Acetyl-leucin; dies entspricht einer Ausbeute von 89,9 %.

Beispiel 11

6,0 g Isovaleraldehyd, 4,13 g Acetamid, 70 ml N-Methylpyrrolidon, 0,25 g Bis(triphenylphosphin)-palladium(II)-chlorid und 2,4 g Natriumbromid werden in einem 150-ml-Autoklaven mit 120 bar CO bei 120°C umgesetzt. Nach einer Reaktionszeit von 60 min wird das Gemisch mittels HPLC analysiert. Man findet 9,86 g N-Acetyl-leucin; dies entspricht einer Ausbeute von 81,3 %.

Beispiel 12

6,0 g Isovaleraldehyd, 4,13 g Acetamid, 70 ml N-Methylpyrrolidon, 0,062 g Palladium(II)-chlorid und 2,1 g Lithiumbromid werden in einem 150-ml-Autoklaven mit 120 bar CO bei 120°C umgesetzt. Nach einer Reaktionszeit von 60 min wird das Gemisch mittels HPLC analysiert. Man findet 9,48 g N-Acetyl-leucin; dies entspricht einer Ausbeute von 78,5 %.

## Patentansprüche

1. Verfahren zur katalytischen Herstellung von N-Acylaminosäuren durch Umsetzung eines phenyl substituierten oder unsubstituierten aliphatischen Aldehyds mit einem aliphatischen oder aromatischen Carbonsäureamid und Kohlenmonoxid in Gegenwart eines dipolaren aprotischen Lösemittels, dadurch gekennzeichnet, daß man als Katalysator ein Gemisch aus einer Palladiumverbindung und einem ionischen Halogenid einsetzt.

2. Verfahren nach Anspruch 1, dadurch gekennzeichnet, daß man die Palladiumverbindung, berechnet auf Palladiummetall, in einer Konzentration von 2 - 10 mmol je Liter Reaktionsgemisch einsetzt.

3. Verfahren nach Anspruch 1 oder 2, dadurch gekennzeichnet, daß man das ionische Halogenid in einer Konzentration von 0,05 - 0,5 mol je Liter Reaktionsgemisch einsetzt.

4. Verfahren nach einem der vorhergehenden Ansprüche, dadurch gekennzeichnet, daß man als ionisches Halogenid ein Bromid einsetzt.

5. Verfahren nach einem der vorhergehenden Anspruche, dadurch gekennzeichnet, daß man als Palladiumverbindung einen Palladium-Phosphin-Komplex einsetzt.

## Claims

1. A process for the catalytic preparation of N-acylamino acids by reacting a phenyl-substituted or unsubstituted aliphatic aldehyde with an aliphatic or aromatic carboxylic acid amide and carbon monoxide in the presence of a dipolar aprotic solvent, which comprises using a mixture of a palladium compound and an ionic halide as the catalyst.

2. The process as claimed in claim 1, which comprises using the palladium compound in a concentration of 2-10 mmol, calculated as palladium metal, per liter of reaction mixture.

3. The process as claimed in claim 1 or 2, which comprises using the ionic halide in a concentration of 0.05 - 0.5 mol per liter of reaction mixture.

4. The process as claimed in any of the preceding claims, which comprises using a bromide as the ionic halide.

5. The process as claimed in any of the preceding claims, which comprises using a palladium-phosphine complex as the palladium compound.

## Revendications

1. Procédé pour la fabrication catalytique de N-acyl-aminoacides par réaction d'un aldéhyde aliphatique phényl-substitué ou non substitué avec un amide d'acide carboxylique aliphatique ou aromatique et le monoxyde de carbone en présence d'un solvant aprotique dipolaire, caractérisé en ce que l'on utilise comme catalyseur un mélange d'un composé de palladium et d'un halogénure ionique.

2. Procédé selon la revendication 1, caractérisé en ce que l'on utilise le composé de palladium, calculé en palladium métal, à une concentration de 2-10 mmol par litre du mélange de réaction.

3. Procédé selon l'une quelconque des revendications précédentes, caractérisé en ce que l'on utilise l'halogénure ionique à une concentration de 0,05-0,5 mol par litre du mélange de réaction.

4. Procédé selon l'une quelconque des revendications précédentes, caractérisé en ce que l'on utilise comme halogénure ionique un bromure.

5. Procédé selon l'une quelconque des revendications précédentes, caractérisé en ce que l'on utilise comme composé de palladium un complexe palladium-phosphine.